# EUROPEAN PATENT APPLICATION

(11) **EP 0 722 931 A1**
(43) Date of publication of application: **24.07.1996**
(21) Application number: 96100752.3
(22) Date of filing: 19.01.1996
(51) Int. Cl.: C07C 68/08, C07C 69/96

(54) **Method for preparing high purity diphenyl carbonate**

(30) Priority: 23.01.1995 JP 8158/95
(71) Applicant: MITSUBISHI GAS CHEMICAL COMPANY, INC., Chiyoda-ku, Tokyo (JP)
(72) Inventor: Takeda, Mutsuhiko, Mitsubishi Gas Chem. Co., Inc., Tsukuba-shi, Ibaraki Pref. (JP); Mizukami, Masamichi, Mitsubishi Gas Chem. Co., Inc, Tsukuba-shi, Ibaraki Pref. (JP); Hirashima Atsushi, Mitsubishi Gas Chem. Co., Inc, Tsukuba-shi, Ibaraki Pref. (JP); Ogi, Hiroaki, Mitsubishi Gas Chem. Co., Inc, Tsukuba-shi, Ibaraki Pref. (JP)
(74) Representative: Goddar, Heinz J., Dr.

(57) **Abstract**

A method for preparing a high purity diphenyl carbonate. The diphenyl carbonate is distilled in the presence of a basic substance. The present invention provides high purity diphenyl carbonate using a simple method. A high molecular aromatic polycarbonate containing substantially no polymerization inhibitor and without coloring is easily prepared from the purified diphenyl carbonate.

## Description

### BACKGROUND OF THE INVENTION

### 1. Field of the Invention

The present invention is directed to a method for preparing a high purity diphenyl carbonate. More particularly, the present invention is directed to a method for preparing a high purity diphenyl carbonate, comprising distilling diphenyl carbonate in the presence of a basic substance. The diphenyl carbonate purified by the method of the present invention does not substantially contain an acidic polymerization inhibitor, and is advantageously used as a monomer component for preparing a polycarbonate made by a transesterification reaction.

### 2. Description of the Related Art

Two known industrial methods for producing an aromatic polycarbonate include (1) a method in which a phosgene and a bisphenol are polymerized at the interface of a two phase dichloromethane/water system in the presence of a hydrogen chloride acceptor (interfacial polymerization method), and (2) a method in which a phenol is removed from a mixture of molten bisphenols and diphenyl carbonate by an transesterification reaction (molten polymerisation method). Today, almost all diphenyl carbonate is produced in solution by the former method.

When a polycarbonate is produced by an interfacial polymerization method, a two-phase dichloromethane-water system is widely used because dichloromethane is an excellent solvent. However, recently, a method not using dichloromethane has been desired due to the resulting problem of water pollution by an alkyl halide, and the molten polymerisation method has attracted the attention of investigators in this field.

In the molten polymerisation method, a polycarbonate is produced by removing phenol from bisphenol and diphenyl carbonate as described above. However, a method of purifying diphenyl carbonate for use as a raw material in a transesterification reaction has not yet been established. Namely, a polycarbonate obtained by the molten polymerisation method is colored, and it is difficult to obtain a product having a high polymerization degree. These are disadvantages in putting the molten polymerization method to practical use.

The principal methods of producing diphenyl carbonate include (1) an aqueous solution method in which phosgene is introduced into an aqueous solution of a metal phenoxide and reacted therewith, (2) an interfacial synthesis method in which the same reaction is conducted in a two phase organic solvent/water system, and (3) a transesterification method which comprises reacting a dialkyl carbonate with phenol. At the present state of the art none of these can be considered exceptional. Therefore, all of these methods are conducted on an industrial scale.

In the aqueous solution method (1), the diphenyl carbonate synthesis reaction does not proceed completely. Consequently, an extra step is needed for removing phenyl chloroformate as an intermediate. In the two phase organic solvent/water system (2), it is impossible to completely remove phenyl chloroformate. Thus, it is also necessary to remove phenyl chloroformate from the product. Furthermore, organic chlorinated impurities included in the phosgene remain in the product, and it is also necessary to remove these impurities. Moreover, when dichloromethane is used as a solvent, the dichloromethane decomposes upon removal from the diphenyl carbonate thus produced. This is due to the presence of a small amount of water which produces a chlorine compound. This in turn causes the problems of apparatus corrosion and product coloration.

When diphenyl carbonate is produced from dimethyl carbonate and phenol by a transesterification method (3), the diphenyl carbonate product becomes contaminated with chlorine. This is due to the copper chloride as a catalyst that is used to prepare the dimethyl carbonate. As a result, purification such as by washing with warm water is required.

Therefore, several methods for purifying diphenyl carbonate have been proposed. For example, Japanese Patent Publication No. 38-1373 discloses treating diphenyl carbonate with warm water or an alkaline aqueous solution to remove phenyl chloroformate remaining in the product. Furthermore, Japanese Patent Publication No. 42-9820 discloses adding a small amount of urea to diphenyl carbonate and heat-melting to remove phenyl chloroformate.

As described above, use of diphenyl carbonate produced according to a conventional method to synthesize polycarbonate results in the problems of coloring and polymerization inhibition. As a result, it is necessary to purify the diphenyl carbonate prior to use as a monomer for producing a polycarbonate, such as by washing with warm water. Several such methods have been proposed, however, the conventional methods are not satisfactory.

### SUMMARY OF THE INVENTION

An object of the present invention is to provide a method for preparing a high purity diphenyl carbonate to thereby remove impurities which cause coloring and inhibit polymerization.

The present inventors have extensively studied the above-described problems, and have obtained an aromatic polycarbonate having a high polymerization activity which is colorless when polymerized, to thereby achieve the present invention.

That is, the above objectives of the present invention have been achieved by providing a method for purifying a high purity diphenyl carbonate which comprises distilling diphenyl carbonate in the presence of a basic substance. In a preferred embodiment, the present invention has been achieved by providing a method for preparing a high purity diphenyl carbonate which comprises contacting diphenyl carbonate prior to distilling with a basic substance at a temperature of 100 °C or more.

The present invention is characterized in that diphenyl carbonate is contacted with a basic substance at a high temperature and then distilled. Therefore, the present invention is essentially different from conventional methods in which diphenyl carbonate is washed with an alkaline aqueous solution. That is, by contacting at a high temperature of 100 °C or more, the washing time which is from 10 to 20 minutes for conventional methods can substantially be made to zero.

Furthermore, a solvent such as water, which have essentially been used in a conventional method, becomes unnecessary. And, an apparatus for purification is also unnecessary. Further, the object of the present invention can be achieved by the extremely simple method in which small amount of a basic substance is added when ordinary distillation is conducted.

Further, as the basic substance is added in the present invention, the reaction rate of the present invention is fast, comparing with the case where urea is simply added. Thus, in the present invention, a problem that when urea, a neutral substance, is added, the reaction rate is slow is solved. And, an apparatus for purification is also unnecessary.

### DETAILED DESCRIPTION OF THE INVENTION

In accordance with the present invention, in spite of a simple method, high purity diphenyl carbonate monomer suitable for producing polycarbonate can be obtained by distilling commercially available diphenyl carbonate under existence of a basic substance. In this case, as mentioned above, purification step is not necessary needed. And, the operation and an apparatus are simple and convenient. The basic substance for use in the present invention is a substance which itself dissociates in aqueous solution, or which dissociates water to produce hydroxyl ion or a proton acceptor. Among these, basic substances which react with diphenyl carbonate to produce a phenoxide are preferred.

Examples of the basic substance include an alkali or alkaline earth metal; an oxide, a hydroxide, a carbonate, a hydride, an amide, an alcoholate and a phenolate of an alkali or alkaline earth metal; a hydride of boron or aluminum; a salt of an organic acid, a phosphate or a borate of an alkali or alkaline earth metal and derivatives thereof; a quaternary ammonium hydroxide; a tertiary amine, etc.

Primary and secondary amines which react with diphenyl carbonate but do not produce a phenoxide are not preferred.

Specific examples of the basic substance for use in the present invention include, for example, alkali and alkaline earth metals such as sodium, potassium and lithium; an oxide, a hydroxide, a carbonate, a hydride, an amide, an alcoholate or a phenolate of an alkali or an alkaline earth metal such as calcium oxide, magnesium oxide, sodium hydroxide, potassium hydroxide, lithium hydroxide, cesium hydroxide, calcium hydroxide, barium hydroxide, magnesium hydroxide, strontium hydroxide, sodium hydrogencarbonate, potassium hydrogencarbonate, sodium carbonate, potassium carbonate, calcium carbonate, sodium hydride, calcium hydride, lithium diisopropyl amide, sodium methoxide, sodium ethoxide, lithium methoxide, lithium ethoxide, potassium methoxide, potassium ethoxide, sodium phenoxide, potassium phenoxide, lithium phenoxide, calcium phenoxide, disodium salt of bisphenol A, dilithium salt of bisphenol A, etc.; hydrides of boron or aluminum such as sodium borohydride, lithium aluminum hydride, tetramethylammonium borohydride, tetrabuthylammonium borohydride, etc.; a salt of an organic acid, or a phosphate or a borate of an alkali or alkaline earth metal and derivatives thereof such as sodium acetate, potassium acetate, lithium acetate, calcium acetate, sodium stearate, potassium stearate, lithium stearate, calcium stearate, sodium benzoate, potassium benzoate, lithium benzoate, disodium hydrogenphosphate, dipotassium hydrogenphosphate, dilithium hydrogenphosphate, disodium phenylphosphate, dipotassium phenylphosphate, dilithium phenylphosphate, sodium borate, etc.; quaternary ammonium hydroxides such as tetramethylammonium hydroxide, tetraethylammonium hydroxide, tetrapropylammonium hydroxide, tetrabuthylammonium hydroxide, benzyltrimethylammonium hydroxide, hexadecyltrimethylammonium hydroxide, etc.; and tertiary amines such as 1-(N,N-bis(2-hydroxyethyl)amino)-2-propanol, 3-(N-benzyl-N-methylamino)-1,2-propanediol, N,N,N',N'-tetrakis(2-hydroxypropyl)ethylenediamine, N-methyldiphenethylamine, triethanolamine, trioctylamine, triphenylamine, etc.

Furthermore, the basic substance for use in the present invention preferably has a boiling point that is higher than diphenyl carbonate. In this manner the basic substance can be removed when the diphenyl carbonate is purified by distillation.

In view of the above, sodium hydroxide, potassium hydroxide, sodium carbonate, potassium carbonate, sodium hydrogencarbonate, potassium hydrogencarbonate, calcium hydride and triethanolamine are particularly preferred as a basic substance.

The basic substance can be added to the diphenyl carbonate either directly or as diluted with an inert solvent such as water. When the basic substance is a liquid or powder at room temperature, a sufficient effect can be obtained even by directly adding the basic substance. However, when the substance is in the form of a solid pellet which is difficult to dissolve in diphenyl carbonate, such as an alkali hydroxide, the basic substance is preferably added as a liquid in order to increase its dispersing quality, although adding as a solid is also effective. When the substance is added with dissolving in an inert solvent, removal of the inert solvent is necessary prior to distilling the diphenyl carbonate.

The addition amount of the basic substance depends on the purity of diphenyl carbonate. Commercially available ordinary diphenyl carbonate contains from about 1 to 10 ppm of chlorine as hydrolytic chlorine (i.e., contains from about 6 to 60 mol ppm of chlorine atom as hydrolytic chlorine).

The polymerization inhibitor or coloring substance that is removed according to the method of the present invention is, mainly a chlorine compound which is derived from hydrolytic chlorine, therefore, the amount of the chlorine compound derived from hydrolytic chlorine can be a standard in outline of addition of a basic substance.

For example, in the case of diphenyl carbonate containing 2.7 ppm of hydrolytic chlorine (i.e., 16.3 mol ppm of hydrolytic chlorine), although a slight improvement can be obtained by adding 10 mol ppm of sodium hydroxide to the diphenyl carbonate, the resulting diphenyl carbonate has poor polymerizability.

Accordingly, the effect of the present invention cannot be achieved by adding a basic substance in about the same molar amount as the hydrolytic chlorine content of the diphenyl carbonate. Rather, it is necessary to add the basic substance in a molar amount of from several times to several tens of times greater than the molar content of hydrolytic chlorine.

Furthermore, the polymerization inhibitor or coloring causative agent is not limited to compounds derived from hydrolytic chlorine. Thus, the addition amount of the basic substance is generally in the range from about 10 to about 1000 mol ppm. However, the amount is preferably determined on a practical basis by preparatory purification and polymerization testing to study the polymerizability and extent of coloring.

The term "ppm" in the present specification means "weight/weight×10⁶", while "mol ppm" means "mol/mol×10⁶".

The term "hydrolytic chlorine" means chlorine that is extractable with water when the diphenyl carbonate is dissolved or in a molten state. More particularly, hydrolytic chlorine means chlorine which is derived from hydrolyzable compounds, such as salts, e.g., sodium chloride, etc.; and phenyl chloroformate. The chlorine can be measured by ion chromatography, once the diphenyl carbonate is dissolved in toluene, etc., and then extracted with warm water.

In the present invention, the distillation technique is not particularly limited. Both single distillation and multi distillation can be used, and in either case a batch or continuous system can be employed. A flash distillation and thin layer-distillation can also be used. Furthermore, the distillation can be separately conducted more than one time. The degree of vacuum is not particularly limited, however, in view of the boiling point of diphenyl carbonate, the distillation is suitably conducted in the range of from 0.01 to 760 mmHg.

In the present invention, the timing of addition of the basic substance and the position of addition in the distillation apparatus is not particularly limited, however, the basic substance is most desirably mixed into the diphenyl carbonate prior to the distillation.

In the present invention, in the distillation of the diphenyl carbonate containing a basic substance using a distillation tower, the temperature inside the distillation tower is at least 100 °C or more. Because the boiling point is usually 100 °C or more when the distillation is conducted at a vacuum pressure of 1 mmHg or more, it is sufficient to continue the distillation under these conditions. However, when the vacuum pressure becomes very small and the temperature inside the distillation tower (inner tower temperature) is 100 °C or less, the diphenyl carbonate mixture containing a basic substance may be heated prior to feeding to the distillation tower.

Furthermore, in one embodiment of the present invention, the diphenyl carbonate and basic substance are mixed and heated prior to distillation and stored. The mixture is then distilled at a later time.

The purified diphenyl carbonate that is obtained by the method of the present invention contains hydrolytic chlorine in an amount of 0.3 ppm or less, and can be used without additional processing as a raw material for producing polycarbonate. Further purification, such as by washing with warm water, is unnecessary. Also, the purified diphenyl carbonate can be used as a raw material for other chemical reactions.

### EXAMPLES

The method of present invention will be explained in more detail in the following Examples. However, the present invention should not be construed as being limited to these Examples.

### Example 1

A thermometer and a magnetic stirrer were installed in a 500 ml flask, and a Vigreaux distilling column, a distilling head, an air cooling apparatus and a receptacle were mounted thereon to make a distillation apparatus.

To this apparatus were added 214.22 g (1.0 mol) of diphenyl carbonate (manufactured by Bayer) containing 2.7 ppm hydrolytic chlorine and 1 ml of 1 N sodium hydroxide (1000 mol ppm based on diphenyl carbonate). The mixture was then heated and distilled at a boiling point of 165 °C/10 mmHg.

After about 6 g (3%) of diphenyl carbonate were removed as a pre-distillate, almost the entire amount (95%) was recovered as purified diphenyl carbonate (95 % recovery). The diphenyl carbonate thus obtained was evaluated by the following method. The results are shown in Table 1.

Hydrolytic chlorine: 5 g of diphenyl carbonate was accurately weighed, and then 10 ml of toluene was added thereto. The mixture was melted at 60 °C using a hot water bath, and then 10 ml of an extractant containing an internal standard (NaBr as an internal standard was added to 2.8 mM of NaHCO₃/ 2.25 mM Na₂CO₃) was added thereto. This mixture was stirred with a magnetic stirrer for 6 hours, while warming with a hot water bath at 60 °C. After the mixture was allowed to cool by standing, the water layer was analyzed by ion chromatography.

Evaluation of polymerizability: A 200 ml glass round flask having a stirring apparatus including a teflon agitating blade fitted to a chrome plated agitating bar was used as a polymerization apparatus. 22.83 g of (0.10 mol) bisphenol A (manufactured by Nippon Steel Chemical Co., Ltd.), 50 µl of an aqueous solution of 0.42 mg/ml of disodium phenylphosphate (9.6×10⁻⁸ mol), and 21.93 g of diphenyl carbonate (0.1024 mol) were added thereto and kept under a nitrogen atmosphere at 180 °C for 13 minutes, followed by agitation at from 200 to 210 rpm. After 15 minutes, the pressure was reduced to 40 mmHg. Then, 2 hours and 15 minutes later, the mixture was heated to thereby increase the temperature at a rate of 0.4 °C/minute. After one hour and 30 minutes from the start of a raise temperature, the pressure was reduced to 30 mmHg. 15 minutes later, the pressure was further reduced to 20 mmHg, and then 15 minutes later the pressure was yet further reduced to 10 mmHg to thereby promote the polymerization reaction. Finally, the pressure was reduced to 0.1 mmHg or less, and the mixture was reacted for an additional 1 hour (the final temperature was 265 °C). The polycarbonate thus obtained was removed and evaluated in terms of molecular weight and color tone by the following method.

Molecular weight: 160 g of the polymerized sample was dissolved in 80 ml of methylene chloride. The viscosity thereof was measured using an ubbelohde viscometer, to thereby obtain the viscosity-average molecular weight.

Color tone (APHA): 4 g of the polymerized sample was dissolved into 25 ml of methylene chloride and compared with a standard liquid.

### Example 2

The same procedures were followed and the resulting product was evaluated as in Example 1, except that 100 mol ppm of an aqueous solution of sodium hydroxide based on diphenyl carbonate, i.e., 1 ml of a 0.1 N sodium hydroxide solution was added as a basic substance. The results are shown in Table 1.

### Example 3

The same procedures were followed and the resulting product was evaluated as in Example 1, except that 10 mol ppm of sodium hydroxide based on diphenyl carbonate, i.e., 1 ml of a 0.01 N sodium hydroxide solution was added as a basic subtance. The results are shown in Table 1.

### Example 4

The same procedures were followed and the resulting product was evaluated as in Example 1, except that 0.0106 g of powdered sodium carbonate (100 mol ppm) was added as a basic substance. The results are shown in Table 1.

### Comparative Example 1

The same procedures were followed and the resulting product was and evaluated as in Example 1, except that an aqueous solution of sodium hydroxide was not added (i.e., the product was not distilled in the presence of a basic substance). The results are shown in Table 1. Although the hydrolytic chlorine content was rather small, polymerizability was hardly improved by distillation.

### Comparative Example 2

To 214.22 g of diphenyl carbonate were added 214.22 g of an alkaline aqueous solution (an alkali concentration of 1000 mol ppm) prepared by adding high purity water to 100 mol ppm of 0.01 N aqueous solution of sodium hydroxide. The mixture was stirred at 90 °C for 30 minutes. After the mixture was allowed to cool by standing, the solid thus obtained was filtered by suction, vacuum-dried, and then evaluated following the procedure of Example 1. The results are shown in Table 1.

### Comparative Example 3

Commercially available diphenyl carbonate was used without distillation purification, and the resulting product was evaluated as in Example 1. The results are shown in Table 1. Although the hydrolytic chlorine content was rather small, polymerizability was hardly improved.

**Table 1**

| | treatment/addition amount of basic substance | Mv (Viscosity-Average Molecular Weight) | APHA | hydrolytic chlorine content |
|---|---|---|---|---|
| Example 1 | NaOH 1000 (mol ppm) | 19500 | 5 | 0.2 ppm |
| Example 2 | NaOH 100 (mol ppm) | 16300 | 5 | 0.3 ppm |
| Example 3 | NaOH 10 (mol ppm) | 8500 | 5 | 0.4 ppm |
| Example 4 | Na₂CO₃ 100 (mol ppm) | 18300 | 5 | 0.1 ppm |
| Comparative Example 1 | distillation only | 2200 | 5-10 | 0.7 ppm |
| Comparative Example 2 | hot water washing | 3500 | 10-15 | 0.5 ppm |
| Comparative Example 3 | untreated | 1400 | 10-15 | 2.7 ppm |

### Example 5

Distillation was conducted using a 16-stage distillation tower in which Sulzer laboratory-scale packing (manufactured by Sumitomo Heavy Industries, Ltd., 55 mm per 1 unit) was packed by five units into an concentration part and a recovery part of a continuous distillation apparatus. The distillation apparatus was provided with a reflux apparatus at the upper part thereof and with a feed portion of a raw material at a central part of the tower.

100 mol ppm of triethanolamine was added to diphenyl carbonate (manufactured by Bayer, containing 1.0 ppm hydrolytic chlorine) and dissolved therein. The continuous distillation was conducted at a reflux ratio of 1 and a tower top pressure of 10 mmHg under the following conditions, the raw material was introduced into the central part of the distillation tower at a rate of 200 g/h. The temperature was controlled such that 190 g/h of diphenyl carbonate was obtained from top of the tower.

At equilibrium, the pot pressure was 17.5 mmHg, the temperature of the pot liquid was 183 °C, and the temperature of the tower top was 168 °C. The distillation was continued under these conditions for 5 hours. The diphenyl carbonate obtained in the last one hour of operation was evaluated in the same manner as in Example 1. The results are shown in Table 1.

### Comparative Example 4

The same purification by distillation was conducted as in Example 5, except that triethanolamine was not added to the diphenyl carbonate. The results are shown in Table 2.

**Table 2**

| | treatment | Mv (Viscosity-Average Molecular Weight) | APH A | hydrolytic chlorine content |
|---|---|---|---|---|
| Example 5 | triethanoamine added | 18500 | 5-10 | 0.2 ppm |
| Comparative Example 1 | continuous distillation only | 4000 | 5-10 | 0.4 ppm |

Thus, the present invention provides a method for purifying diphenyl carbonate to a high level of purity using a simple method. The thus obtained diphenyl carbonate is advantageously used to prepare polycarbonate having a high polymerization degree without coloring.

It should further be apparent to those skilled in art that various changes in form and detail of the invention as shown and described above may be made. It is intended that such changes be included within the spirit and scope of the claims appended hereto.

## Claims

1. A method for preparing a purity diphenyl carbonate which comprises distilling diphenyl carbonate in the presence of a basic substance.

2. The method of claim 1, wherein said distilling comprises distilling in a distillation column having an inner tower temperature of at least 100 °C.

3. A method for preparing a purity diphenyl carbonate which comprises contacting diphenyl carbonate prior to distilling with a basic substance at a temperature of 100 °C or more.

4. The method of claim 1, wherein the basic substance is selected from the group consisting of an alkali metal or alkaline earth metal; an oxide, a hydroxide, a carbonate, a hydride, an amide, an alcoholate or a phenolate of an alkali or alkaline earth metal; a hydride of boron or aluminum; a salt of an organic acid; a phosphate or borate of an alkali or alkaline earth metal; a quaternary ammonium hydroxide; and a tertiary amine.

5. The method of claim 2, wherein the basic substance is selected from the group consisting of an alkali metal or alkaline earth metal; an oxide, a hydroxide, a carbonate, a hydride, an amide, an alcoholate or a phenolate of an alkali or alkaline earth metal; a hydride of boron or aluminum; a salt of an organic acid, a phosphate or a borate of an alkali or alkaline earth metal; a quaternary ammonium hydroxide; and a tertiary amine.

6. The method of claim 3, wherein the basic substance is selected from the group consisting of an alkali metal or alkaline earth metal; an oxide, a hydroxide, a carbonate, a hydride, an amide, an alcoholate or a phenolate of an alkali or alkaline earth metal; a hydride of boron or aluminum; a salt of an organic acid; a phosphate or borate of an alkali or alkaline earth metal; a quaternary ammonium hydroxide; and a tertiary amine.

7. The method of claim 1, wherein the basic substance is selected from the group consisting of sodium hydroxide, potassium hydroxide, sodium carbonate, potassium carbonate, sodium hydrogencarbonate, potassium hydrogencarbonate, calcium hydroxide and triethanolamine.

8. The method of claim 2, wherein the basic substance is selected from the group consisting of sodium hydroxide, potassium hydroxide, sodium carbonate, potassium carbonate, sodium hydrogencarbonate, potassium hydrogencarbonate, calcium hydroxide and triethanolamine.

9. The method of claim 3, wherein the basic substance is selected from the group consisting of sodium hydroxide, potassium hydroxide, sodium carbonate, potassium carbonate, sodium hydrogencarbonate, potassium hydrogencarbonate, calcium hydroxide and triethanolamine.

10. The method of claim 1, wherein said basic substance is present in an amount of from 10 to 1000 mols per 10⁶ mols of diphenyl carbonate.

11. The method of claim 2, wherein said basic substance is present in an amount of from 10 to 1000 mols per 10⁶ mols of diphenyl carbonate.

12. The method of claim 3, wherein said basic substance is present in an amount of from 10 to 1000 mols per 10⁶ mols of diphenyl carbonate.
